# EUROPEAN PATENT APPLICATION

(11) **EP 3 805 258 A1**
(43) Date of publication of application: **14.04.2021**
(21) Application number: 19812551.0
(22) Date of filing: 30.05.2019
(51) Int. Cl.: C07K 14/705, A61K 47/68, A61P 27/14, A61P 31/00, A61P 35/00, A61P 37/04, A61P 37/06, A61K 38/00

(54) **PHARMACEUTICAL COMPOSITION FOR PREVENTING OR TREATING CANCER, CONTAINING CD300E INHIBITOR AS ACTIVE INGREDIENT**

(30) Priority: 30.05.2018 KR 20180062068
(71) Applicant: Genome and Company, Seongnam-si, Gyeonggi-do 13486 (KR)
(72) Inventor: YOON, Kyoung Wan, Seongnam-si, Gyeonggi-do 13486 (KR); HOUH, Youn Kyung, Seongnam-si, Gyeonggi-do 13486 (KR); JEON, Bu-Nam, Seongnam-si, Gyeonggi-do 13486 (KR); SOHN, Jinyoung, Seongnam-si, Gyeonggi-do 13486 (KR); KIM, Yun Yeon, Seongnam-si, Gyeonggi-do 13486 (KR); LEE, Suro, Seongnam-si, Gyeonggi-do 13486 (KR); CHUNG, Joo-Yeon, Seongnam-si, Gyeonggi-do 13486 (KR); JEONG, Areum, Seongnam-si, Gyeonggi-do 13486 (KR)
(74) Representative: Dehns
(86) International application number: PCT/KR2019/006479
(87) International publication number: WO 2019/231247

(57) **Abstract**

The present invention relates to a pharmaceutical composition for preventing or treating cancer, containing a CD300E inhibitor as an active ingredient. The CD300E inhibitor according to the present invention can increase the activity of immunocytes so as to be usable as an adjuvant. In addition, the CD300E inhibitor according to the present invention enhances the immunity of an individual, thereby enabling cancer to be effectively prevented or treated.

<110>     genomeandcompany

<120>     PHARMACEUTICAL COMPOSITION FOR PREVENTING OR TREATING CANCER
          COMPRISING CD300E INHIBITOR AS ACTIVE INGREDIENT

<130>     PCB903026GNC

<150>     KR 10-2018-0062068
<151>     2018-05-30

<160>     18

<170>     KoPatentIn 3.0

<210>     1
<211>     205
<212>     PRT
<213>     Artificial Sequence

<220>
<223>     CD300E(full length)


<400>     1

<210>    2
<211>    618
<212>    DNA
<213>    Artificial Sequence

<220>
<223>    CD300E(full length)

<400>    2
atgtggctgc tcccagctct actccttctc tgcctctcag gctgtttgtc tctgaagggc    60

cccggctctg tgactggcac tgcgggggac tctctgacag tgtggtgtca gtatgagagc    120

atgtacaagg gatataacaa gtactggtgc cgaggacagt acgacacgtc atgtgagagc    180

attgtggaga ccaagggaga agagaaggtg gagaggaatg gccgcgtgtc catcagagac    240

cacccggagg ctctcgcctt cactgtgacc atgcagaacc tcaatgaaga tgatgctgga    300

tcttactggt gcaaaattca gacagtgtgg gtcctggatt catggtcacg cgatccctcg    360

gacctggtta gggtgtatgt ttccccagca attacaaccc caaggaggac cacacatcca    420

gccacacctc ccatcttcct ggtggtgaac cctgggcgaa acctcagcac cagggaggtg    480

ttgacccaaa attcagggtt ccggctcagc agccctcact tcctgctcgt ggtccttctg    540

aagctgcctc tgctcctgag catgctgggt gctgttttct gggtgaacag gcctcagtgg    600

gctcctcctg gaagatag                                                  618

<210>    3
<211>    173
<212>    PRT
<213>    Artificial Sequence

<220>
<223>    CD300E extracellular domain

<400>    3
Met Trp Leu Leu Pro Ala Leu Leu Leu Leu Cys Leu Ser Gly Cys Leu
1               5                   10                  15

Ser Leu Lys Gly Pro Gly Ser Val Thr Gly Thr Ala Gly Asp Ser Leu
            20                  25                  30

Thr Val Trp Cys Gln Tyr Glu Ser Met Tyr Lys Gly Tyr Asn Lys Tyr
        35                  40                  45

Trp Cys Arg Gly Gln Tyr Asp Thr Ser Cys Glu Ser Ile Val Glu Thr
    50                  55                  60

Lys Gly Glu Glu Lys Val Glu Arg Asn Gly Arg Val Ser Ile Arg Asp
65                  70                  75                  80

His Pro Glu Ala Leu Ala Phe Thr Val Thr Met Gln Asn Leu Asn Glu
                85                  90                  95

Asp Asp Ala Gly Ser Tyr Trp Cys Lys Ile Gln Thr Val Trp Val Leu

            100                 105                 110

Asp Ser Trp Ser Arg Asp Pro Ser Asp Leu Val Arg Val Tyr Val Ser
        115                 120                 125

Pro Ala Ile Thr Thr Pro Arg Arg Thr Thr His Pro Ala Thr Pro Pro
    130                 135                 140

Ile Phe Leu Val Val Asn Pro Gly Arg Asn Leu Ser Thr Arg Glu Val
145                 150                 155                 160

Leu Thr Gln Asn Ser Gly Phe Arg Leu Ser Ser Pro His
                165                 170

<210> 4
<211> 519
<212> DNA
<213> Artificial Sequence

<220>
<223> CD300E extracellular domain

<400> 4
atgtggctgc tcccagctct actccttctc tgcctctcag gctgtttgtc tctgaagggc 60

cccggctctg tgactggcac tgcgggggac tctctgacag tgtggtgtca gtatgagagc 120

atgtacaagg gatataacaa gtactggtgc cgaggacagt acgacacgtc atgtgagagc 180

attgtggaga ccaagggaga agagaaggtg gagaggaatg gccgcgtgtc catcagagac 240

cacccggagg ctctcgcctt cactgtgacc atgcagaacc tcaatgaaga tgatgctgga 300

tcttactggt gcaaaattca gacagtgtgg gtcctggatt catggtcacg cgatccctcg 360

gacctggtta gggtgtatgt ttccccagca attacaaccc caaggaggac cacacatcca 420

gccacacctc ccatcttcct ggtggtgaac cctgggcgaa acctcagcac cagggaggtg 480

ttgacccaaa attcagggtt ccggctcagc agccctcac 519

<210> 5
<211> 285
<212> PRT
<213> Artificial Sequence

<220>
<223> human CD300E with Human IgG constant kappa tag

<400> 5
Met Trp Leu Leu Pro Ala Leu Leu Leu Leu Cys Leu Ser Gly Cys Leu
1               5                   10                  15

Ser Leu Lys Gly Pro Gly Ser Val Thr Gly Thr Ala Gly Asp Ser Leu
            20                  25                  30

Thr Val Trp Cys Gln Tyr Glu Ser Met Tyr Lys Gly Tyr Asn Lys Tyr
        35                  40                  45

Trp Cys Arg Gly Gln Tyr Asp Thr Ser Cys Glu Ser Ile Val Glu Thr
50                  55                  60

Lys Gly Glu Glu Lys Val Glu Arg Asn Gly Arg Val Ser Ile Arg Asp
65                  70                  75                  80

His Pro Glu Ala Leu Ala Phe Thr Val Thr Met Gln Asn Leu Asn Glu
                85                  90                  95

Asp Asp Ala Gly Ser Tyr Trp Cys Lys Ile Gln Thr Val Trp Val Leu
            100                 105                 110

Asp Ser Trp Ser Arg Asp Pro Ser Asp Leu Val Arg Val Tyr Val Ser
        115                 120                 125

Pro Ala Ile Thr Thr Pro Arg Arg Thr Thr His Pro Ala Thr Pro Pro
    130                 135                 140

Ile Phe Leu Val Val Asn Pro Gly Arg Asn Leu Ser Thr Arg Glu Val
145                 150                 155                 160

Leu Thr Gln Asn Ser Gly Phe Arg Leu Ser Ser Pro His Gly Gln Ala
                165                 170                 175

Gly Gln Arg Thr Val Ala Ala Pro Ser Val Phe Ile Phe Pro Pro Ser
            180                 185                 190

Asp Glu Gln Leu Lys Ser Gly Thr Ala Ser Val Val Cys Leu Leu Asn
        195                 200                 205

Asn Phe Tyr Pro Arg Glu Ala Lys Val Gln Trp Lys Val Asp Asn Ala
    210                 215                 220

Leu Gln Ser Gly Asn Ser Gln Glu Ser Val Thr Glu Gln Asp Ser Lys
225                 230                 235                 240

Asp Ser Thr Tyr Ser Leu Ser Ser Thr Leu Thr Leu Ser Lys Ala Asp
                245                 250                 255

Tyr Glu Lys His Lys Val Tyr Ala Cys Glu Val Thr His Gln Gly Leu
            260                 265                 270

Ser Leu Pro Val Thr Lys Ser Phe Asn Arg Gly Glu Ser
        275                 280                 285


<210>    6
<211>    858
<212>    DNA
<213>    Artificial Sequence

<220>
<223>    human CD300E with Human IgG constant kappa tag


<400>    6
atgtggctgc tcccagctct actccttctc tgcctctcag gctgtttgtc tctgaagggc        60

cccggctctg tgactggcac tgcgggggac tctctgacag tgtggtgtca gtatgagagc       120

atgtacaagg gatataacaa gtactggtgc cgaggacagt acgacacgtc atgtgagagc       180

attgtggaga ccaagggaga agagaaggtg gagaggaatg gccgcgtgtc catcagagac       240

cacccggagg ctctcgcctt cactgtgacc atgcagaacc tcaatgaaga tgatgctgga 300

tcttactggt gcaaaattca gacagtgtgg gtcctggatt catggtcacg cgatccctcg 360

gacctggtta gggtgtatgt ttccccagca attacaaccc caaggaggac cacacatcca 420

gccacacctc ccatcttcct ggtggtgaac cctgggcgaa acctcagcac cagggaggtg 480

ttgacccaaa attcagggtt ccggctcagc agccctcacg gccaggccgg ccagcgaact 540

gtggctgcac catctgtctt catcttcccg ccatctgatg agcagttgaa atctggaact 600

gcctctgttg tgtgcctgct gaataacttc tatcccagag aggccaaagt acagtggaag 660

gtggataacg ccctccaatc gggtaactcc caggagagtg tcacagagca ggacagcaag 720

gacagcacct acagcctcag cagcaccctg acgctgagca aagcagacta cgagaaacac 780

aaagtctacg cctgcgaagt cacccatcag ggcctgagct tgcccgtcac aaagagcttc 840

aacaggggag agtcttga 858

<210> 7
<211> 19
<212> RNA
<213> Artificial Sequence

<220>
<223> human CD300E siRNA-sense(Group 5)

<400> 7
cagcauagga agaacucua 19

<210> 8
<211> 19
<212> RNA
<213> Artificial Sequence

<220>
<223> human CD300E siRNA-antisense(Group 5)

<400> 8
uagaguucuu ccuaugcug 19

<210> 9
<211> 19
<212> RNA
<213> Artificial Sequence

<220>
<223> human CD300E siRNA-sense(Group 6)

<400> 9
gagaagucaa gagccauga 19

<210>    10
<211>    19
<212>    RNA
<213>    Artificial Sequence

<220>
<223>    human CD300E siRNA-antisense(Group 6)


<400>    10
ucauggcucu ugacuucuc                                                    19


<210>    11
<211>    19
<212>    RNA
<213>    Artificial Sequence

<220>
<223>    human CD300E siRNA-sense(Group 7)


<400>    11
ugacagugug gugucagua                                                    19


<210>    12
<211>    19
<212>    RNA
<213>    Artificial Sequence

<220>
<223>    human CD300E siRNA-antisense(Group 7)


<400>    12
uacugacacc acacuguca                                                    19


<210>    13
<211>    19
<212>    RNA
<213>    Artificial Sequence

<220>
<223>    mouse CD300E siRNA-sense(Group 8)


<400>    13
gugucaauau aguccauca                                                    19


<210>    14
<211>    19
<212>    RNA
<213>    Artificial Sequence

<220>
<223>    mouse CD300E siRNA-antisense(Group 8)


<400>    14
ugauggacua uauugacac                                                    19

<210>	15
<211>	19
<212>	RNA
<213>	Artificial Sequence

<220>
<223>	mouse CD300E siRNA-sense(Group 9)


<400>	15
gaggauuguu ccaccauca	19


<210>	16
<211>	19
<212>	RNA
<213>	Artificial Sequence

<220>
<223>	mouse CD300E siRNA-antisense(Group 9)


<400>	16
ugauggugga acaauccuc	19


<210>	17
<211>	19
<212>	RNA
<213>	Artificial Sequence

<220>
<223>	mouse CD300E siRNA-sense(Group 10)


<400>	17
acgugaucca ucggucagu	19


<210>	18
<211>	19
<212>	RNA
<213>	Artificial Sequence

<220>
<223>	mouse CD300E siRNA-antisense(Group 10)


<400>	18
acugaccgau ggaucacgu	19

## Description

### Technical Field

The present invention relates to a pharmaceutical composition for preventing or treating cancer, comprising a CD300E inhibitor as an active ingredient.

### Background Art

T cells play an important role in immunity of the human body. T cells are divided into killer T cells, helper T cells, regulatory T cells, and memory T cells. In particular, killer T cells express CD8 on the cell surface; helper T cells express CD4 on the cell surface; and regulatory T cells express CD4 and CD25 on the cell surface.

When an antigen such as a bacterium enters from the outside, helper T cells secrete substances such as cytokines to activate killer T cells and B cells. The activated killer T cells kill pathogen-infected cells, and the activated B cells secrete antibodies to inhibit activity of the antigen. Recently, attempts have been made to treat diseases such as cancer by activating such immunoregulatory abilities of T cells.

In addition, a T cell-mediated disease is recognized as a disease representing various immune-system diseases. In particular, T cells are considered as causing and perpetuating autoimmune diseases. Immune responses to self-antigens are caused by continuous or periodic activation of autoreactive T cells. In addition, the autoreactive T cells are attracting attention as a cause for characteristic tissue injury and tissue destruction which are directly or indirectly identified in autoimmune diseases.

Meanwhile, programmed cell death ligand 1 (PD-L1) is a type 1 transmembrane protein which is a ligand for programmed cell death-1 (PD-1). PD-L1 is expressed in hematopoietic cells such as T lymphocytes, B lymphocytes, dendritic cells, or macrophages. PD-1 is known as an immune checkpoint factor or immunomodulator which regulates secondary signaling activity of T cells. In addition, it has been reported that PD-1 is capable of acting to inhibit functions of T cells, such as inhibiting proliferation of T cells and decreasing expression of cytokines, by binding to PD-L1 or the like which is expressed on the surface of cells such as activated T cells or dendritic cells (Krzysztof M. Zak, et al., 2015).

Recently, attempts have been made to develop anticancer agents and immunomodulators using substances that regulate immune functions of T cells, such as PD-1 and PD-L1.

### Disclosure of Invention

### Technical Problem

In the course of studying substances capable of inhibiting or increasing activity of immune cells, the present inventors have found that CD300E cell signaling system can regulate activity of T cells, and thus have completed the present invention.

### Solution to Problem

In an embodiment, there is provided an immunopotentiator comprising, as an active ingredient, a substance that binds to CD300E protein or a substance that inhibits expression of CD300E gene.

In addition, in an embodiment, there is provided a pharmaceutical composition for preventing or treating cancer, comprising, as an active ingredient, a substance that binds to CD300E protein or a substance that inhibits expression of CD300E gene.

In addition, in an embodiment, there is provided a method for treating cancer, comprising a step of administering the pharmaceutical composition to an individual.

### Advantageous Effects of Invention

The CD300E inhibitor according to the present invention can increase activity of immune cells, and thus can be used as an immunopotentiator. In addition, the CD300E inhibitor according to the present invention can potentiate immunity of an individual, and thus can be used to effectively prevent or treat cancer.

### Brief Description of Drawings

Fig. 1 illustrates a proliferation rate (%) of CD4+ T cells, inhibited by CD300E protein.
Fig. 2 illustrates a proliferation rate (%) of CD8+ T cells, inhibited by CD300E protein.
Figs. 3a to 3d illustrate cytotoxicity (%) of peripheral blood mononuclear cells (PBMCs) per group, obtained when the lung cancer cell line A549 and PBMCs are treated with a CD300E inhibitor (antibody or siRNA).
Figs. 4a to 4d illustrate cytotoxicity (%) of PBMCs per group, obtained in the case where the colon cancer cell line HCT-116 and PBMCs are treated with a CD300E inhibitor (antibody or siRNA).
Figs. 5a to 5d illustrate cytotoxicity (%) of PBMCs per group, obtained in the case where the breast cancer cell line MDA-MB-231 and PBMCs are treated with a CD300E inhibitor (antibody or siRNA).
Figs. 6a to 6d illustrate cytotoxicity (%) of PBMCs per group, obtained in the case where the gastric cancer cell line MKN-74 and PBMCs are treated with a CD300E inhibitor (antibody or siRNA).
Figs. 7a to 7d illustrate cytotoxicity (%) of PBMCs per group, obtained in the case where the blood cancer cell line U937 and PBMCs are treated with a CD300E inhibitor (antibody or siRNA).
Figs. 8a to 8d illustrate changes in tumor size in mice treated with a CD300E inhibitor (each of 3 types of siRNAs), per group.

### Best Mode for Carrying out the Invention

In an embodiment, there is provided an immunopotentiator comprising, as an active ingredient, a substance that binds to CD300 antigen like family member E (CD300E) protein or a substance that inhibits expression of CD300E gene.

As used herein, the term "CD300E" is the abbreviation of " CD300 antigen like family member E", and may be a protein having the amino acid sequence of SEQ ID NO: 1. The polypeptide having the amino acid sequence of SEQ ID NO: 1 may be encoded by a polynucleotide having the base sequence of SEQ ID NO: 2.

Specifically, the sequence of 205 amino acids represented by SEQ ID NO: 1 may be comprised of an extracellular domain consisting of amino acids at positions 1 to 173 (SEQ ID NO: 3), a transmembrane domain consisting of amino acids at positions 174 to 194, and an intracellular domain consisting of amino acids at positions 195 to 205. The extracellular domain of CD300E having the amino acid sequence of SEQ ID NO: 3 may be encoded by a polynucleotide having the base sequence of SEQ ID NO: 4.

In addition, in the present disclosure, CD300E may be in a form in which a human IgG constant kappa tag is bound. The CD300E to which the human IgG constant kappa tag is bound for isolation and purification may be represented by the amino acid sequence of SEQ ID NO: 5, and the polypeptide having the amino acid sequence of SEQ ID NO: 5 may be encoded by a polynucleotide having the base sequence of SEQ ID NO: 6. Specifically, the fusion protein of SEQ ID NO: 5 may be a protein in which the extracellular domain of CD300E (at positions 1 to 173) and the human IgG constant kappa tag (at positions 179 to 285) are bound to each other.

In the present disclosure, the substance that binds to CD300E protein may be a compound, an aptamer, a peptide, or an antibody or a fragment thereof which specifically binds to the CD300E protein. The antibody or a fragment thereof may be any one selected from the group consisting of a monoclonal antibody, scFv, Fab, Fab', and F(ab)'.

In the present disclosure, the substance that inhibits expression of CD300E gene may be an antisense nucleic acid, siRNA, shRNA, miRNA, or ribozyme which complementarily binds to DNA or mRNA of the CD300E gene. The CD300E siRNA may be any one of the base sequences of SEQ ID NOS: 7 to 18.

In one embodiment, it was intended to identify whether an anti-CD300E antibody or CD300E siRNA, which targets CD300E and inhibits activity thereof, can increase cytotoxicity of peripheral blood mononuclear cells (PBMCs) against cancer cells. As a result, in a mixture of PBMCs and a cancer cell line which had been treated with a CD300E inhibitor, cytotoxicity of the PBMCs against the cancer cell line was exhibited at a higher level than a control group. In another embodiment of the present disclosure, it was intended to identify whether CD300E siRNA that inhibits activity of CD300E inhibits growth of tumors in mice. As a result, in mice in which CD300E had been knocked down by treatment with CD300E siRNA, the tumor growth rate was remarkably inhibited as compared with a control group. From the above results, it is found that the anti-CD300E antibody or CD300E siRNA, which targets CD300E and inhibits activity thereof, can delay or stop the progress of cancer by blocking or knocking down CD300E to inhibit activity or expression thereof.

In addition, in an embodiment, there is provided a pharmaceutical composition for preventing or treating cancer, comprising, as an active ingredient, a substance that binds to CD300E protein or a substance that inhibits expression of CD300E gene.

The substance that binds to CD300E protein or the substance that inhibits expression of CD300E gene is as described above. The pharmaceutical composition may further comprise a pharmaceutically acceptable additive.

In the present disclosure, the cancer may be, but is not limited to, any one selected from the group consisting of bladder cancer, bone cancer, blood cancer, breast cancer, melanoma, thyroid cancer, parathyroid cancer, bone marrow cancer, rectal cancer, throat cancer, laryngeal cancer, lung cancer, esophageal cancer, pancreatic cancer, colorectal cancer, gastric cancer, tongue cancer, skin cancer, brain tumor, uterine cancer, head or neck cancer, gallbladder cancer, oral cancer, colon cancer, perianal cancer, central nervous system tumor and liver cancer.

A preferred daily dose of the pharmaceutical composition for preventing or treating cancer, comprising, as an active ingredient, a substance that binds to CD300E protein or a substance that inhibits expression of CD300E gene according to the present disclosure may be in a range of 0.01 ug/kg to 10 g/kg and preferably 0.01 mg/kg to 1 g/kg, depending on the patient's condition, body weight, sex, age, severity of disease, and route of administration. Administration may be carried out once or several times a day.

In addition, in an embodiment, there is provided a method for treating cancer, comprising a step of administering, to an individual, a pharmaceutical composition for preventing or treating cancer, comprising, as an active ingredient, a substance that binds to CD300E protein or a substance that inhibits expression of CD300E gene.

The route of administration may be, but is not limited to, any one selected from the group consisting of intravenous, intramuscular, intradermal, subcutaneous, intraperitoneal, intraarteriole, intraventricular, intralesional, intrathecal, topical, and combinations thereof.

### Mode for the Invention

Hereinafter, the present invention will be described in more detail by way of the following examples. However, the following examples are intended to only illustrate the present invention, and the scope of the present invention is not limited thereto.

### Preparation Example 1. Preparation of buffers

Buffers used in the examples were prepared as follows:
IX PBS (Thermo Fisher gibco #10010) was prepared by mixing 155 mM sodium chloride, 2.96 mM sodium phosphate solution, and 1.05 mM potassium phosphate solution, pH 7.4.

FACS buffer was prepared by mixing IX PBS (Thermo Fisher gibco #10010), 10 ml of 2% FBS (Thermo Fisher gibco #16000-044), and 1 ml of 1 mM EDTA (Fisher 15575020).

IX RBC lysis buffer was prepared by diluting 10X RBC solution (Biolegend #420301) in triple-distilled water at 1:10.

10% FBS RPMI1640 was prepared by mixing RPMI medium (Cellgrow #10-040-CVR), 50 ml of 10% FBS (Thermo Fisher gibco #16000-044), 5 ml of 1% antibiotics (Thermo Fisher gibco #15140-122), and 0.5 ml of 2-mercaptoethanol (Thermo Fisher gibco #21985-023).

MACS buffer was prepared by mixing IX PBS (Thermo Fisher gibco #10010), 0.5% bovine serum albumin (BSA) (Millipore #82-100-6), and 2 ml of 2 mM EDTA (Fisher 15575020).

### Example 1. Identification of inhibitory effect of CD300E protein on proliferation and activity of T cells

In the present example, it was intended to identify whether CD300E protein inhibits proliferation and activity of T cells, and thus causes cancer cells to avoid the T cell-mediated immune system.

### Example 1.1. Preparation of CD4+ T cells and CD8+ T cells

Human blood was collected and placed in a 10-mL tube coated with EDTA (or heparin). The human blood was mixed with PBS at a ratio of 1:1. Then, Ficoll-Paque PLUS was placed in a 50-mL tube, and the above blood sample was added thereto. After centrifugation, human PBMCs were collected. The collected product was centrifuged to remove the supernatant. Subsequently, RBC lysis (1x) buffer was added thereto, pipetting was performed, and then the resultant was kept on ice for 3 minutes. Subsequently, 50 ml of 10% FBS RPMI1640 was added thereto and the mixture was centrifuged to remove supernatant. Then, FACS buffer was added thereto and centrifugation was performed to remove the supernatant. Thereafter, 50 ml of MACS buffer (PBS containing 0.5% BSA and 2 mM EDTA) was added thereto, the number of cells was counted, and centrifugation was performed to remove the supernatant.

CD4+ T cells and CD8+ T cells were resuspended using 40 µl of MACS buffer per 1 × 10⁷ cells. 10 µl of each of anti-CD4 and anti-CD8 biotin antibodies was placed in the tube, and then the tube was kept in a refrigerator for 5 minutes. Thereafter, 30 µl of MACS buffer per 1 × 10⁷ cells was added to the resulting product. 20 µl of anti-biotin microbeads was added thereto and mixing was performed. Subsequently, CD4+ T cells and CD8+ T cells were isolated using LS column and the number of the respective cells was counted.

Each of the prepared CD4+ T cells and CD8+ T cells was mixed with 1 µl of carboxyfluorescein succinimidyl ester (CFSE) per 2 × 10⁶ cells and each mixture was kept at 37°C for 3 minutes. Then, FBS was added to each tube containing each of CD4+ T cells and CD8+ T cells, and each tube was kept on ice for 10 minutes. Thereafter, centrifugation was performed to remove the supernatant. 30 ml of FACS buffer was added to the resulting product, and then pipetting was performed. Centrifugation was performed to remove the supernatant. Then, 10% FBS RPMI1640 was added thereto, and then pipetting was performed. Centrifugation was performed to remove the supernatant. Thereafter, the resulting product was mixed with 10 ml of 10% FBS RPMI1640, and then the number of cells was counted.

### Example 1.2. Identification of inhibited activity of T cells caused by CD300E protein

Recombinant human IgG1 protein (Cat. No. 110-HG) and recombinant human PD-L1/B7-H1 protein (Cat. No. 156-B7) were purchased from R&D Systems. Recombinant human CD300E protein (human CD300E with human IgG constant kappa tag) was produced by the following method. Specifically, using pCEP4 (Invitrogen) plasmid DNA as a vector, a polynucleotide represented by SEQ ID NO: 6 was cloned into the multiple cloning site (MCS) region thereof. The resulting DNA construct was transfected into human embryonic kidney (HEK) 293F cells, and the recombinant human CD300E protein (human CD300E with human IgG constant kappa tag) contained in the culture medium was purified and obtained using kappa select resin.

7.5 µg/ml or 10 µg/ml of each of the proteins was mixed with 2.5 µg/ml of anti-CD3 antibody (BioLegend, Cat. No. 317325). Each of the mixtures was used to coat a 96-well plate at 4°C, and washing was performed three times with PBS. The CD4+ T cells prepared in Example 1.1 were added at 200 µl in an amount of 2 × 10⁶ cells per each well of the 96-well plate and incubation was performed. Activation of the CD4+ T cells using the anti-CD3 antibody was allowed to occur for 72 hours.

In addition, 5 µg/ml or 7.5 µg/ml of each of the proteins was mixed with 2.5 µg/ml of anti-CD3 antibody (BioLegend, Cat. No. 317325). Each of the mixtures was used to coat a 96-well plate at 4°C, and washing was performed three times with PBS. The CD8+ T cells prepared in Example 1.1 were added at 200 µl in an amount of 2 × 10⁶ cells per each well of the 96-well plate and incubation was performed. Activation of the CD8+ T cells using the anti-CD3 antibody was allowed to occur for 72 hours.

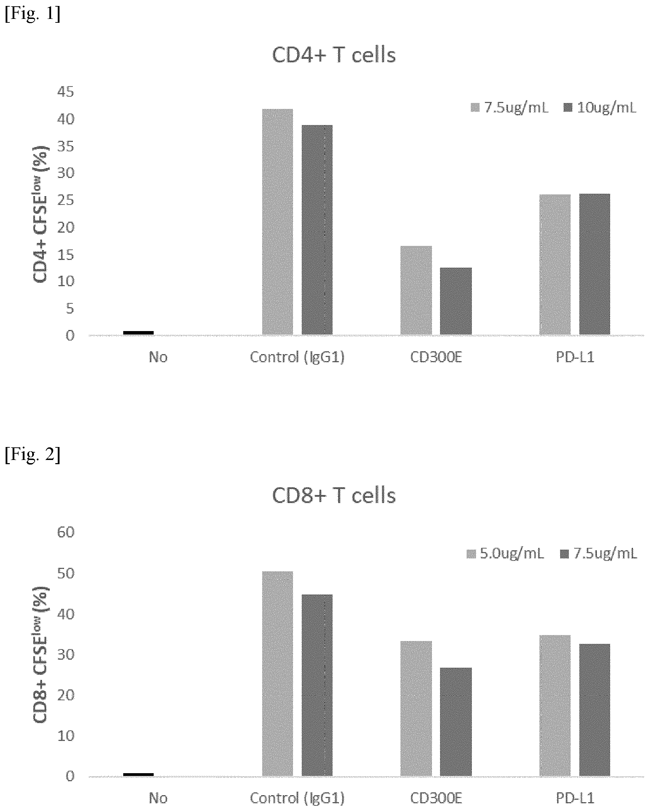

Here, proliferation of the CD4+ T cells and the CD8+ T cells was measured by a level of CFSE staining, and was analyzed by flow cytometry using FACSDiVa software (BD Biosciences). The results are illustrated in Figs. 1 and 2.

Figs. 1 and 2 are bar graphs representing proliferation rates (%) of the CD4+ T cells and the CD8+ T cells measured by flow cytometry. As illustrated in Figs. 1 and 2, in the control group treated with PD-L1, proliferation of both the CD4+ T cells and the CD8+ T cells was inhibited as compared with the control group treated with IgG1.

In addition, in the group treated with CD300E, proliferation of the CD4+ T cells and the CD8+ T cells was remarkably inhibited as compared with the control group treated with IgG1, and proliferation of the CD4+ T cells and the CD8+ T cells was inhibited even as compared with the control group treated with PD-L1. From these results, it can be seen that neutralization of CD300E due to its blocking or knockdown decreases an inhibitory ability of CD300E on proliferation of T cells, and thus enables effective treatment of cancer.

### Example 2. PBMC cytotoxicity assay

In the present example, it was intended to identify whether PBMCs are capable of exhibiting increased cytotoxicity (killing ability) against cancer cells when CD300E is neutralized using a CD300E inhibitor.

### Example 2.1. Preparation of PBMCs

Human blood was collected and placed in a 10-mL tube coated with EDTA (or heparin). The human blood was mixed with PBS at a ratio of 1:1. Then, Ficoll-Paque PLUS was placed in a 50-mL tube, and the above blood sample was added thereto. After centrifugation, human PBMCs were collected.

1.0 µg/ml of anti-CD3 antibody (BioLegend, Cat. No. 317325) was used to coat a 96-well plate at 4°C. Each well of the 96-well plate was washed 3 times with PBS before addition of the PBMCs. The previously obtained PBMCs were mixed with 10% FBS RPMI1640 and added at 100 µl in an amount of 6 × 10⁵ cells per each well of the 96-well plate. Activation of the PBMCs by the anti-CD3 antibody was allowed to occur for 72 hours.

### Example 2.2. Preparation of cancer cells

The lung cancer cell line A549 (ATCC®CCL-185), the colon cancer cell line HCT-116 (ATCC®CCL-247), the breast cancer cell line MDA-MB-231 (ATCC®HTB-26), the gastric cancer cell line MKN-74 (KCLB No. 80104), and the leukemia cell line U937 (ATCC®CRL-1593.2) were each mixed with 5 µM of CFSE and kept at 37°C for 5 minutes. Thereafter, FBS was added to each tube containing each cell line, and each tube was kept on ice for 10 minutes. Subsequently, centrifugation was performed to remove the supernatant. To the product thus obtained was added 30 ml of FACS buffer. Then, pipetting was performed and centrifugation was performed to remove the supernatant. Then, 10% FBS RPMI1640 was added thereto. Then, pipetting was performed and centrifugation was performed to remove the supernatant. The product thus obtained was mixed with 10 ml of 10% FBS RPMI1640, and then the number of cells was counted.

Each type of the cancer cells was added at 3 × 10⁴ cells/100 µl per each PBMC-containing well of the 96-well plate prepared in Example 2.1, and incubation was performed.

### Example 2.3. Measurement of cytotoxicity of PBMCs against cancer cell lines

10 µg/mL of anti-human CD300E antibody or 50 nM CD300E siRNA was added to each well of the 96-well plate, and incubation was performed for 24 hours. Table 1 below shows experimental groups, in which four types of neutralizing antibodies are used to block CD300E, and an untreated control group; and Table 2 below shows experimental groups, in which three types of siRNAs are used to knock down CD300E, and an untreated control group.

**[Table 1]**

| | Human CD300E neutralizing antibody |
|---|---|
| Control group | Untreated |
| Group 1 | anti-human CD300E antibody (LifeSpan BioSciences, Inc., LS-C159307) |
| Group 2 | anti-human CD300E antibody (GeneTex, GTX34214) |
| Group 3 | anti-human CD300E antibody (R&D Systems, MAB2705) |
| Group 4 | anti-human CD300E antibody (R&D Systems, AF2705) |

**[Table 2]**

| | Human CD300E siRNA |
|---|---|
| Control group | Untreated |
| Group 5 | Sense (5'-CA GCA UAG GAA GAA CUC UA-3') (SEQ ID NO: 7) |
| | Antisense (5'-UA GAG UUC UUC CUA UGC UG-3') (SEQ ID NO: 8) |
| Group 6 | Sense (5'-GA GAA GUC AAG AGC CAU GA-3') (SEQ ID NO: 9) |
| | Antisense (5'-UC AUG GCU CUU GAC UUC UC-3') (SEQ ID NO: 10) |
| Group 7 | Sense (5'-UG ACA GUG UGG UGU CAG UA-3') (SEQ ID NO: 11) |
| | Antisense (5'-UA CUG ACA CCA CAC UGU CA-3') (SEQ ID NO: 12) |

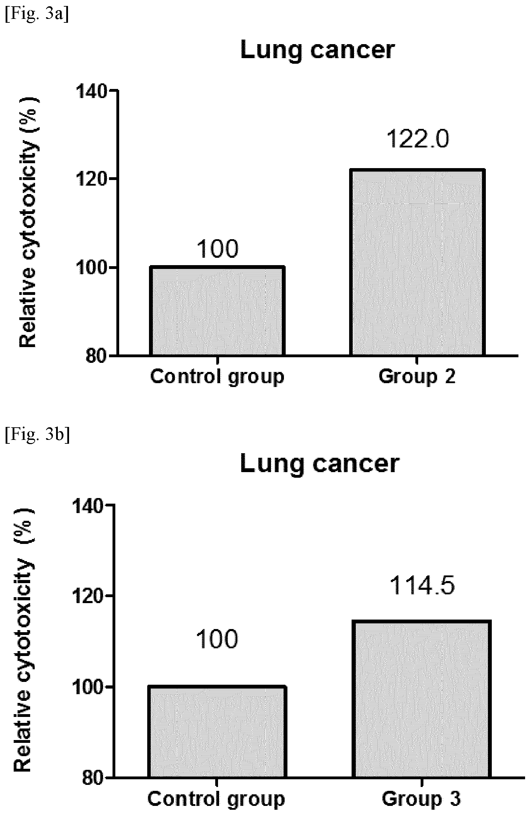

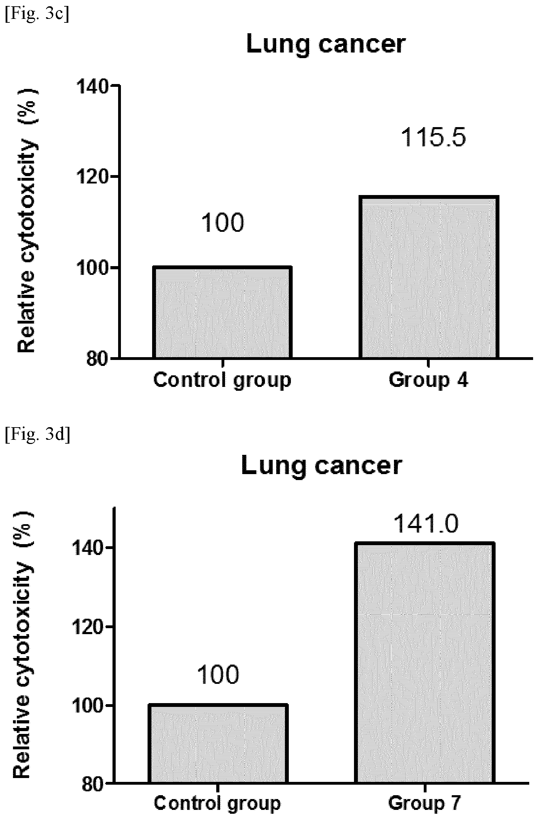

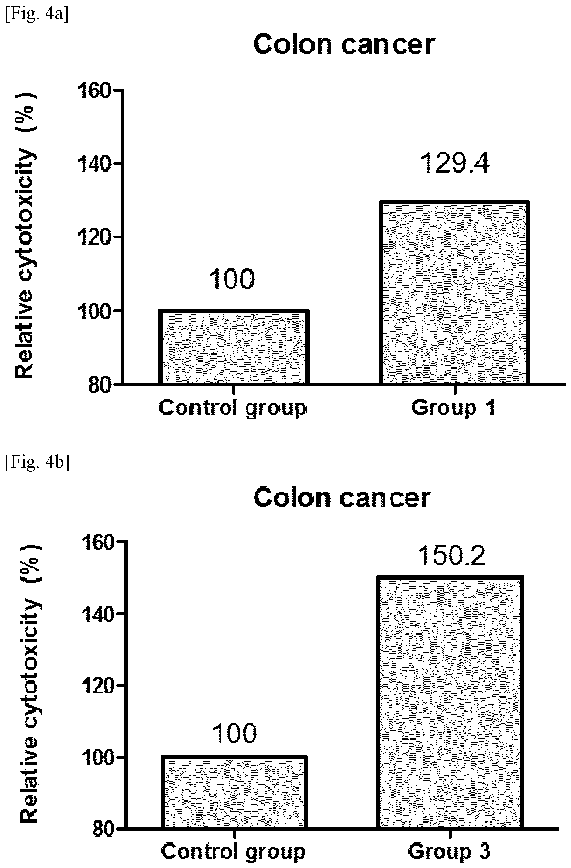

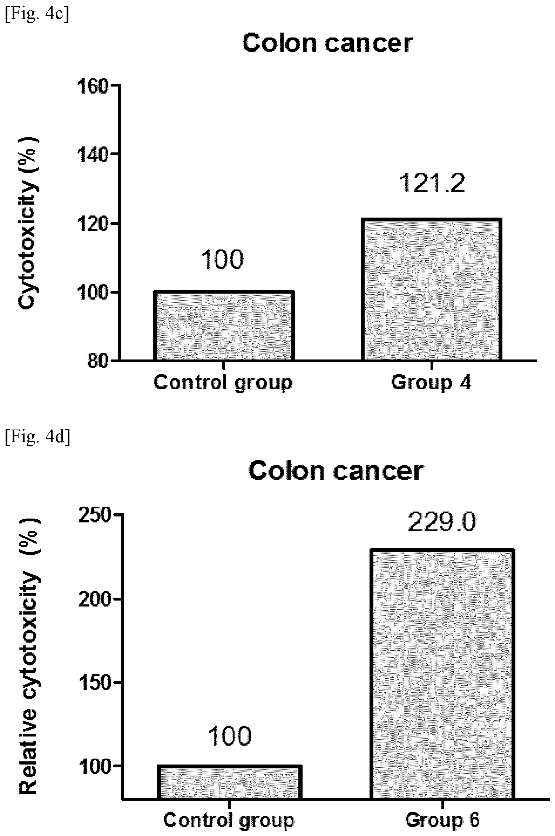

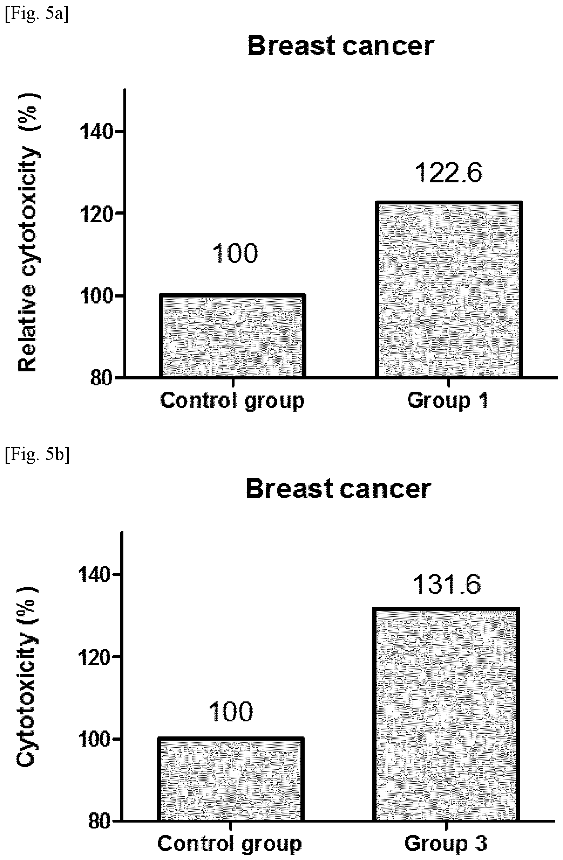

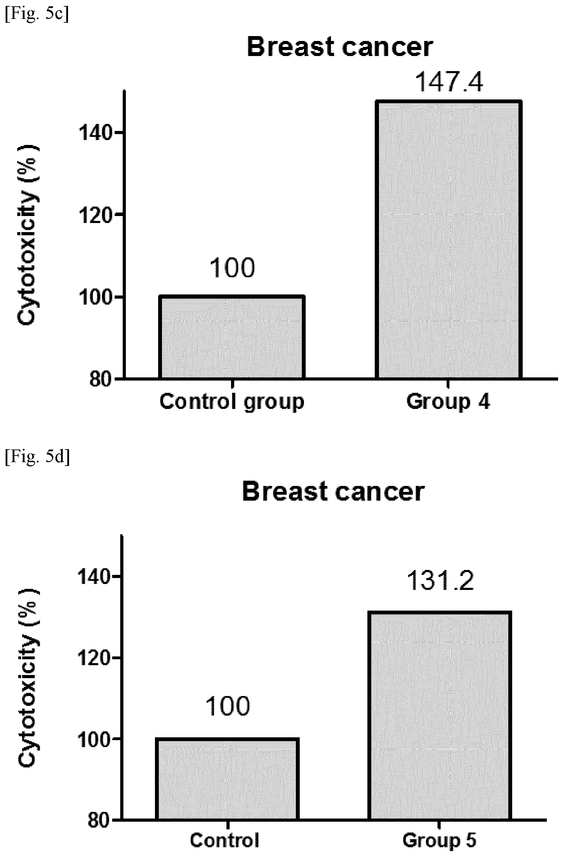

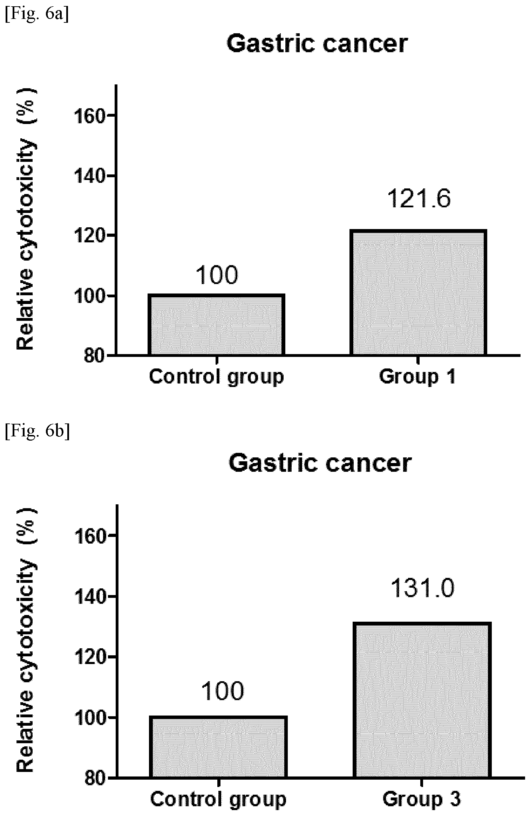

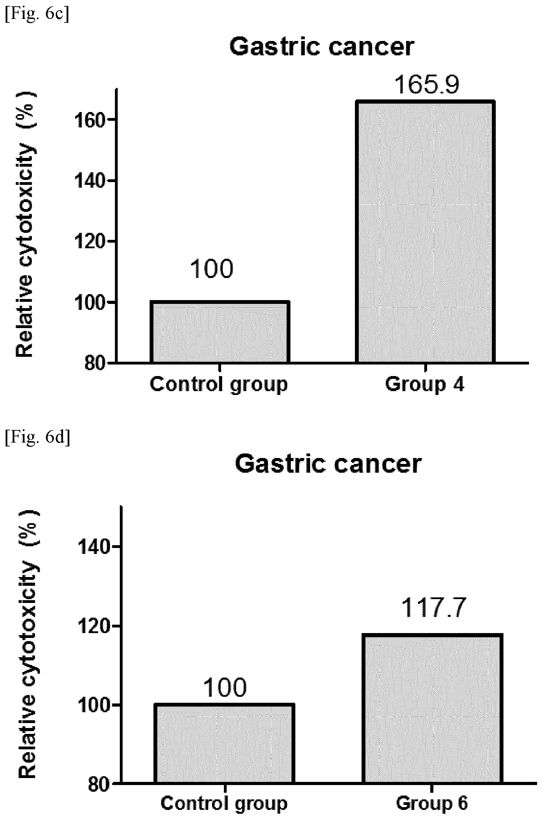

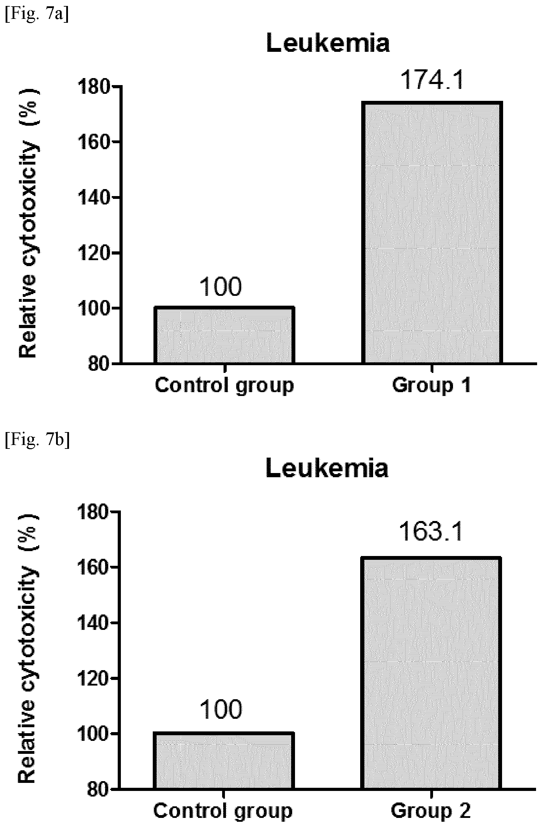

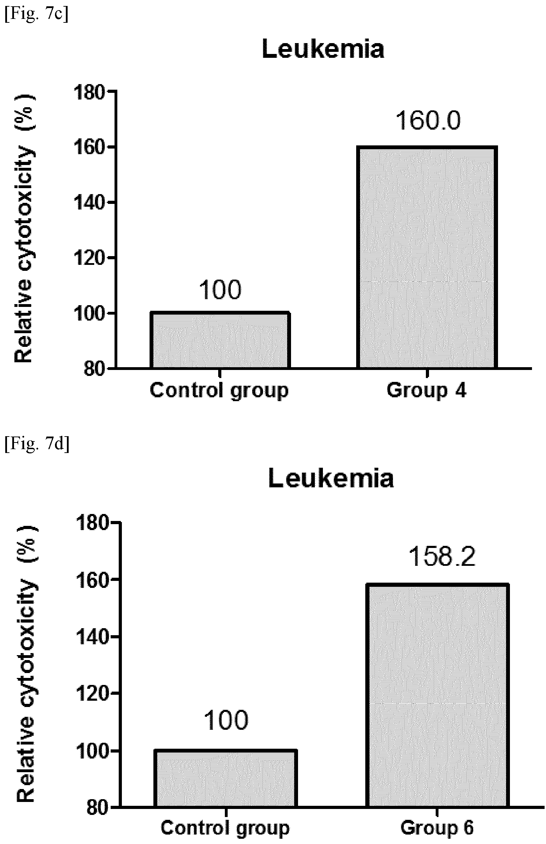

Each mixture of PBMCs and each cancer cell line was incubated with the antibody or siRNA. After 24 hours, in order to identify lysed cells, the cells were stained with 7-aminoactinomycin D (7-AAD; BD Pharmingen, San Diego, Calif., USA). Staining for CFSE and 7-AAD was measured using the FACSDiVa software (BD Biosciences), to identify the PBMC's cytolytic ability against each cancer cell line. The results are illustrated in Figs. 3a to 7d. Specifically, the experimental results obtained by treating the lung cancer cell line A549 with the CD300E neutralizing antibody or siRNA are illustrated in Figs. 3a to 3d. The experimental results obtained by treating the colon cancer cell line HCT-116 with the CD300E neutralizing antibody or siRNA are illustrated in Figs. 4a to 4d. The experimental results obtained by treating the breast cancer cell line MDA-MB-231 is treated with the CD300E neutralizing antibody or siRNA are illustrated in Figs. 5a to 5d. The experimental results obtained by treating the gastric cancer cell line MKN-74 with the CD300E neutralizing antibody or siRNA are illustrated in Figs. 6a to 6d. The experimental results obtained by treating the leukemia cell line U937 with the CD300E neutralizing antibody or siRNA are illustrated in Figs. 7a to 7d.

As illustrated in Figs. 3a to 3d, in the case where the lung cancer cell line A549 and PBMCs are treated with the CD300E neutralizing antibody, a significantly increased lung cancer cell-killing ability was exhibited as compared with the untreated control group, although there was a difference in degree, in terms of the killing ability, depending on types of antibodies; and even in the case where the lung cancer cell line is treated with CD300E siRNA, a significantly increased lung cancer cell-killing ability was also exhibited.

Similar to the above results that PBMCs exhibit an increased killing ability against the lung cancer cell line, it was found that PBMCs also exhibit an increased killing ability against the colon cancer cell line, the breast cancer cell line, the gastric cancer cell line, and the leukemia cell line in the case where CD300E is neutralized using the CD300E neutralizing antibody or siRNA (Figs. 4a to 7d).

### Example 3. Experiments using tumor mouse model

In the present example, it was intended to identify *in vivo* whether growth of tumors in mice is inhibited when CD300E is neutralized using a CD300E inhibitor.

The MC38 cell line derived from C57BL/6 colon adenocarcinoma cells was resuspended at a concentration of 2.0 × 10⁵ cells in 50 µl of PBS and subcutaneously injected into the flank of 6-week-old female C57BL/6 mice. Table 3 below shows experimental groups, in which siRNA is used to knock down CD300E, and an untreated control group.

**[Table 3]**

| | Mouse CD300E siRNA |
|---|---|
| Control group | Untreated |
| Group 8 | Sense (5'-GU GUC AAU AUA GUC CAU CA-3') (SEQ ID NO: 13) |
| | Antisense (5'-UG AUG GAC UAU AUU GAC AC-3') (SEQ ID NO: 14) |
| Group 9 | Sense (5'-GA GGA UUG UUC CAC CAU CA-3') (SEQ ID NO: 15) |
| | Antisense (5'-UG AUG GUG GAA CAA UCC UC-3') (SEQ ID NO: 16) |
| Group 10 | Sense (5'-AC GUG AUC CAU CGG UCA GU-3') (SEQ ID NO: 17) |
| | Antisense (5'-AC UGA CCG AUG GAU CAC GU-3') (SEQ ID NO: 18) |

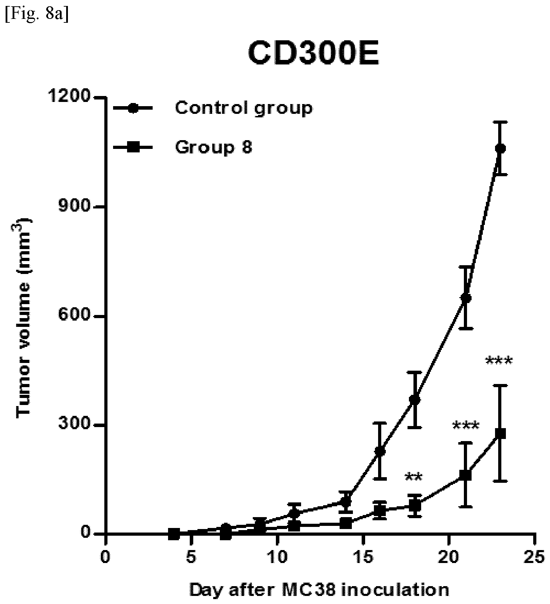

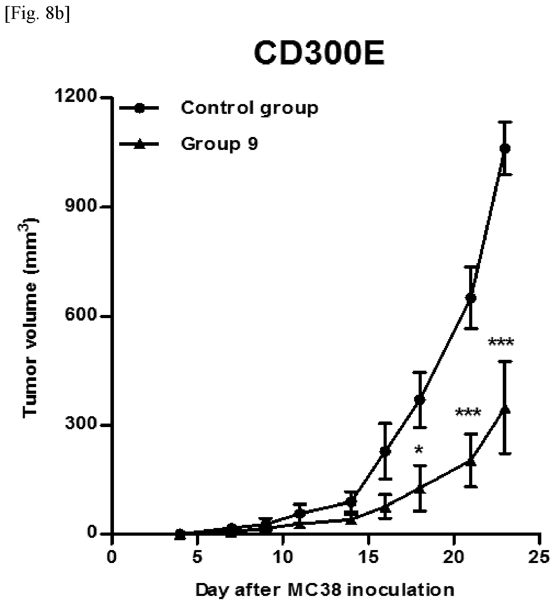

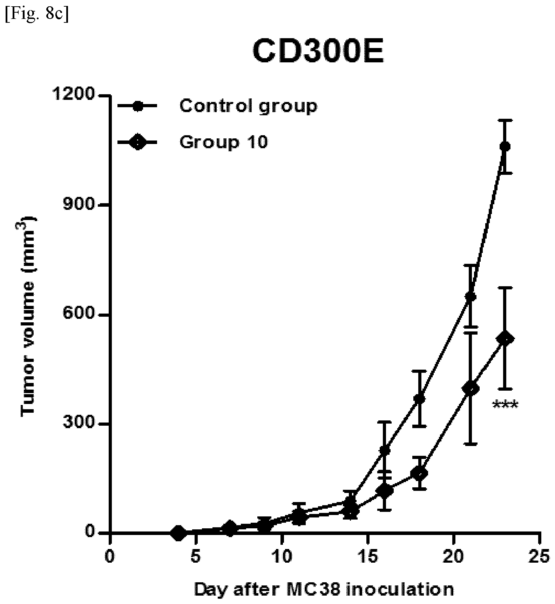

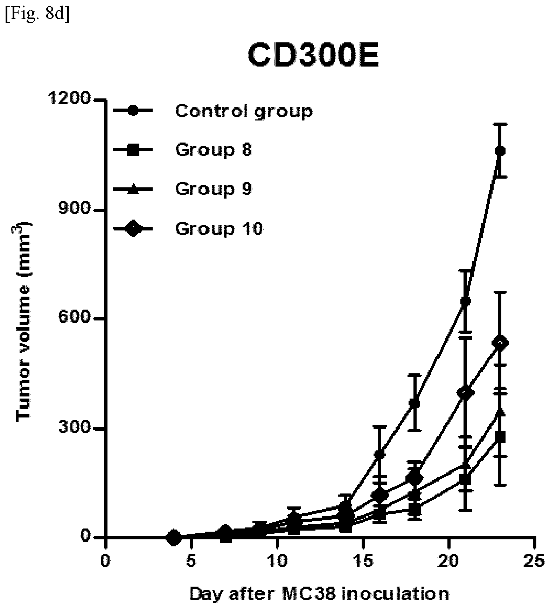

For all experimental groups, starting from the 11^{th} day after injection of the MC38 cell line, each siRNA targeting mouse CD300E was injected into the mouse tumor three times in total at intervals of 5 days. Specifically, 10 µg of siRNA was mixed with 7.5 µl of Oligofectamine (Invitrogen) in PBS according to the manufacturer's instructions, and then the mixture was injected, at a dose of 0.5 mg/kg, directly into the tumor tissue induced in mice. The results obtained by measuring tumor size in mice in the untreated control group and the experimental groups, in which CD300E is knocked down, are illustrated in Figs. 8a to 8d. As illustrated in Figs. 8a to 8d, it was found that in the untreated control group, the tumor has continuously grown since its generation in mice. On the other hand, it was found that in mice in which CD300E is knocked down, the tumor exhibits a remarkably inhibited growth rate as compared with the untreated control group. This shows that in a case where CD300E is blocked or knocked down and activity or expression thereof is inhibited, progress of cancer is delayed or stopped, and development of cancer is inhibited. Accordingly, a CD300E inhibitor is useful to prevent and treat cancer.

## Claims

1. An immunopotentiator, comprising as an active ingredient:
a substance that binds to CD300 antigen like family member E (CD300E) protein; or
a substance that inhibits expression of CD300E gene.

2. The immunopotentiator of claim 1, wherein the substance that binds to CD300E protein is a compound, an aptamer, a peptide, or an antibody or a fragment thereof which specifically binds to the CD300E protein.

3. The immunopotentiator of claim 2, wherein the CD300E protein has the amino acid sequence of SEQ ID NO: 1 or 3.

4. The immunopotentiator of claim 2, wherein the antibody or a fragment thereof is any one selected from the group consisting of a monoclonal antibody, scFv, Fab, Fab', and F(ab)'.

5. The immunopotentiator of claim 1, wherein the substance that inhibits expression of CD300E gene is an antisense nucleic acid, siRNA, shRNA, miRNA, or ribozyme which complementarily binds to DNA or mRNA of the CD300E gene.

6. The immunopotentiator of claim 5, wherein the DNA of the CD300E gene has the base sequence of SEQ ID NO: 2 or 4.

7. The immunopotentiator of claim 5, wherein the siRNA is any one of the base sequences of SEQ ID NOS: 7 to 18.

8. A pharmaceutical composition for preventing or treating cancer, comprising as an active ingredient:
a substance that binds to CD300E protein; or
a substance that inhibits expression of CD300E gene.

9. The pharmaceutical composition of claim 8, wherein the substance that binds to CD300E protein is a compound, an aptamer, a peptide, or an antibody or a fragment thereof which specifically binds to the CD300E protein.

10. The pharmaceutical composition of claim 9, wherein the CD300E protein has the amino acid sequence of SEQ ID NO: 1 or 3.

11. The pharmaceutical composition of claim 9, wherein the antibody or a fragment thereof is any one selected from the group consisting of a monoclonal antibody, scFv, Fab, Fab', and F(ab)'.

12. The pharmaceutical composition of claim 8, wherein the substance that inhibits expression of CD300E gene is an antisense nucleic acid, siRNA, shRNA, miRNA, or ribozyme which complementarily binds to DNA or mRNA of the CD300E gene.

13. The pharmaceutical composition of claim 12, wherein the DNA of the CD300E gene has the base sequence of SEQ ID NO: 2 or 4.

14. The pharmaceutical composition of claim 12, wherein the siRNA is any one of the base sequences of SEQ ID NOS: 7 to 18.

15. The pharmaceutical composition of claim 8, wherein the cancer is any one selected from the group consisting of bladder cancer, bone cancer, blood cancer, breast cancer, melanoma, thyroid cancer, parathyroid cancer, bone marrow cancer, rectal cancer, throat cancer, laryngeal cancer, lung cancer, esophageal cancer, pancreatic cancer, colorectal cancer, gastric cancer, tongue cancer, skin cancer, brain tumor, uterine cancer, head or neck cancer, gallbladder cancer, oral cancer, colon cancer, perianal cancer, central nervous system tumor and liver cancer.

16. A method for treating cancer, comprising:
administering, to an individual, the pharmaceutical composition of any one of claims 8 to 15.
